# EUROPÄISCHE PATENTSCHRIFT

(11) **EP 2 279 121 B1**
(45) Veröffentlichungstag und Bekanntmachung des Hinweises auf die Patenterteilung: **23.05.2012**
(21) Anmeldenummer: 09733027.8
(22) Anmeldetag: 16.02.2009
(51) Int. Cl.: B65B 3/30, B65B 3/36, B65B 39/00, A61M 39/28, G05D 7/01, B67C 3/28

(54) **EINWEG-FÜLLVORRICHTUNG**
DISPOSABLE FILLING APPARATUS
DISPOSITIF DE REMPLISSAGE A USAGE UNIQUE

(30) Priorität: 16.04.2008 DE 102008001204
(43) Veröffentlichungstag der Anmeldung: 02.02.2011
(73) Patentinhaber: Robert Bosch GmbH, 70442 Stuttgart (DE)
(72) Erfinder: MUELLER, Frank, 73479 Ellwangen (DE); BRAEUNINGER, Marc, 74589 Satteldorf (DE); MAYER, Werner, 74599 Wallhausen (DE)
(86) Internationale Anmeldenummer: PCT/EP2009/051766
(87) Internationale Veröffentlichungsnummer: WO 2009/127454

(56) Entgegenhaltungen:
- EP-A- 1 930 241
- DE-A1- 10 152 234
- DE-A1- 19 951 555
- DE-A1-102005 008 041
- DE-U1- 8 335 564
- US-A- 3 265 249
- US-A- 4 570 822
- US-A- 5 549 672

## Beschreibung

### Stand der Technik

Die vorliegende Erfindung betrifft eine Einweg-Füllvorrichtung zum Abfüllen einer definierten Menge eines Fluids in einen Behälter, insbesondere bei pharmazeutischen Anwendungen.

In der Pharmaindustrie müssen verschiedene, insbesondere flüssige Wirkstoffe in definierten Mengen mit engen Toleranzen im Behälter, wie z.B. Fläschchen o.ä., abgefüllt werden. Hierzu sind Füllsysteme bekannt, welche einen Edelstahlbehälter aufweisen, in welchem das abzufüllende Gut eingefüllt wird. Der Edelstahlbehälter wird verschlossen und durch eine druckbeaufschlagte Leitung oder einen Produkttank gefüllt und sein Inneres mit einem sterilen Gas mit Druck beaufschlagt. Eine Leitung führt vom Edelstahlbehälter zu einem Verteilerrohr, an welchem eine oder mehrere Abfülleinrichtungen in Form von Schläuchen, an deren Ende jeweils eine Füllnadel angeordnet ist, vorgesehen sind. Im Schlauch ist ferner eine Drossel angeordnet, durch welche der abzufüllende Wirkstoff hindurchgeführt wird. Die Drosseln müssen dabei je nach Anwendung ausgewählt werden und sind Bauteile mit engsten Toleranzen, was die Herstellung derartiger Drosseln verteuert. Um eine genaue Abfüllung zu ermöglichen, muss ein Einlern-Vorgang ausgeführt werden, bei dem bei einer konstanten Füllzeit in einem festgelegten Druckbereich mehrere Messpunkte angefahren werden und die dosierte Füllmenge, z.B. mittels einer Waage, ermittelt wird. Aus diesen Punkten wird dann eine Funktion der Füllmenge über dem Druck erstellt, die im Füllsystem hinterlegt wird. Dieses Einlernen muss jedoch für jedes Produkt separat neu ausgeführt werden. Ferner muss bei einem Produktwechsel eine aufwendige Reinigung der Bestandteile des Füllsystems, insbesondere des Edelstahlbehälters und des Verteilerrohrs, erfolgen. Hierzu wird eine Reinigungsflüssigkeit durch das Füllsystem hindurchgeführt, wobei die Reinigung, insbesondere nach Abfüllen von toxischen Produkten, mehrere Stunden dauern kann.

Die DE 199 51555 A1 und die DE 101 52 234 A1 zeigen Füllvorrichtungen mit nichtflexiblen Behältnissen, welche nicht als Einwegartikel vorgesehen sind. Ferner zeigt die DE-U-8335564 ein Infusionsgerät zur Dosierung eines Medikaments oder dgl. zu einem Patienten.

### Vorteile der Erfindung

Die erfindungsgemäße Füllvorrichtung mit den Merkmalen des Patentanspruchs 1 hat demgegenüber den Vorteil, dass sie sehr kostengünstig bereitgestellt werden kann. Ferner entfällt erfindungsgemäß auch eine aufwendige Reinigung des Füllsystems. Die Füllvorrichtung gemäß der vorliegenden Erfindung umfasst dabei einen Beutel, eine Füllnadel und einen den Beutel mit der Füllnadel verbindenden Schlauch, wobei diese Bauteile der Füllvorrichtung eine Baugruppe bilden, die als Einweg-Artikel ausgebildet ist. Nach Beendigung eines Abfüllvorgangs kann die gesamte Baugruppe dann ohne weitere Reinigung entsorgt werden. Die erfindungsgemäße Füllvorrichtung hat ferner den Vorteil, dass sie als vorsterilisiertes System lieferbar ist, so dass die Füllvorrichtung vor einem Abfüllen auch nicht sterilisiert bzw. autoklaviert werden muss. Ferner können insbesondere der Schlauch und die Füllnadel schon individuell auf das abzufüllende Produkt ausgelegt werden, so dass eine höhere Genauigkeit der abgefüllten Mengen erreicht wird. Weiter ist es erfindungsgemäß nicht notwendig, dass die Füllvorrichtung eine Drossel aufweist. Ferner umfasst die Füllvorrichtung ein geschlossenes Druckbehältnis, in welchem der Beutel angeordnet ist. Ein Innenbereich des Druckbehältnisses ist dabei mit Druck beaufschlagbar, um den Beutel bzw. das im Beutel befindliche Produkt unter Druck zu setzen. Hierdurch kann ein einfaches und sicheres Abfüllen des Produktes ermöglicht werden, insbesondere, da das Druckmedium im Druckbehältnis nicht mit dem Produkt in Kontakt kommt. Dadurch ist es nicht notwendig, dass als Druckmedium beispielsweise steril gefiltertes oder sterilisiertes Gas verwendet wird, wie dies im Stand der Technik notwendig ist, da dort das Druckgas direkt mit dem Produkt im Edelstahlbehälter in Kontakt kommt.

Die Unteransprüche zeigen bevorzugte Weiterbildungen der Erfindung.

Weiter bevorzugt umfasst die Füllvorrichtung einen Drucksensor, welcher am Schlauch angeordnet ist, um einen Druck im Schlauch zu bestimmen. Mit Hilfe des Drucksensors kann dabei insbesondere eine Regelung der Füllmenge, vorzugsweise durch eine Änderung eines Durchlassquerschnitts im Bereich des Absperrventils, erfolgen.

Besonders bevorzugt ist am Schlauch eine Membran angeordnet, deren erste Membranfläche sich mit dem im Schlauch geförderten Produkt in Kontakt befindet, und
deren zweite Membranfläche mit dem Drucksensor im Kontakt ist. Hierdurch kann über die Membran der Druck des Produkts im Schlauch bestimmt werden, wobei der Drucksensor schnell und einfach an der Membran befestigt werden kann bzw. wieder von der Membran gelöst werden kann. Der Drucksensor ist dabei kein Einwegartikel, sondern wird mehrfach verwendet.

Weiter bevorzugt ist das Absperrventil der Füllvorrichtung eine Schlauchquetsche, welche von außen am Schlauch angeordnet ist. Der Schlauch ist aus einem flexiblen Material, wie z.B. Silikon, hergestellt und das Absperrventil hat die Aufgabe, den Schlauchdurchmesser vollständig zu verschließen oder zu öffnen.

Gemäß einer weiteren bevorzugten Ausgestaltung der Erfindung umfasst die Füllvorrichtung ein Verzweigungselement, welches im Schlauch angeordnet ist und von welchem mehrere Einzelschläuche aus abgehen, um gleichzeitig mehrere Behälter zu füllen. Das Verzweigungselement ist somit über ein vorzugsweise kurzes Schlauchstück mit dem Beutel verbunden und vom Verzweigungselement gehen dann mehrere Einzelschläuche ab. Dadurch kann insbesondere eine Ausbringungsmenge pro Zeiteinheit vervielfacht werden.

Ferner betrifft die vorliegende Erfindung eine Füllmaschine zur Abfüllung insbesondere pharmazeutischer Produkte, insbesondere pharmazeutischen Flüssigkeiten, welche eine erfindungsgemäße Füllvorrichtung sowie eine Behälterzufuhreinrichtung zum Zuführen von Behältern, in welche das Produkt abgefüllt werden soll, umfasst.

### Zeichnung

Nachfolgend werden bevorzugte Ausführungsbeispiele der Erfindung unter Bezugnahme auf die begleitende Zeichnung im Detail beschrieben. In der Zeichnung ist:
- Figur 1: eine schematische Ansicht einer Füllvorrichtung gemäß einem ersten Ausführungsbeispiel der Erfindung,
- Figur 2: eine schematische Ansicht einer Füllvorrichtung gemäß einem zweiten Ausführungsbeispiel nicht beansprucht,
- Figur 3: eine schematische Ansicht einer Füllvorrichtung gemäß einem dritten Ausführungsbeispiel nicht beansprucht,
- Figur 4: eine schematische Ansicht einer Füllvorrichtung gemäß einem vierten Ausführungsbeispiel nicht beansprucht,
- Figur 5: eine schematische Ansicht einer Füllvorrichtung gemäß einem fünften Ausführungsbeispiel nicht beansprucht,
- Figur 6: eine schematische Ansicht eines in den Füllvorrichtungen verwendbaren Beutels, und
- Figur 7: eine schematische Ansicht eines alternativen in den Füllvorrichtungen verwendbaren Beutels.

### Bevorzugten Ausführungsformen der Erfindung

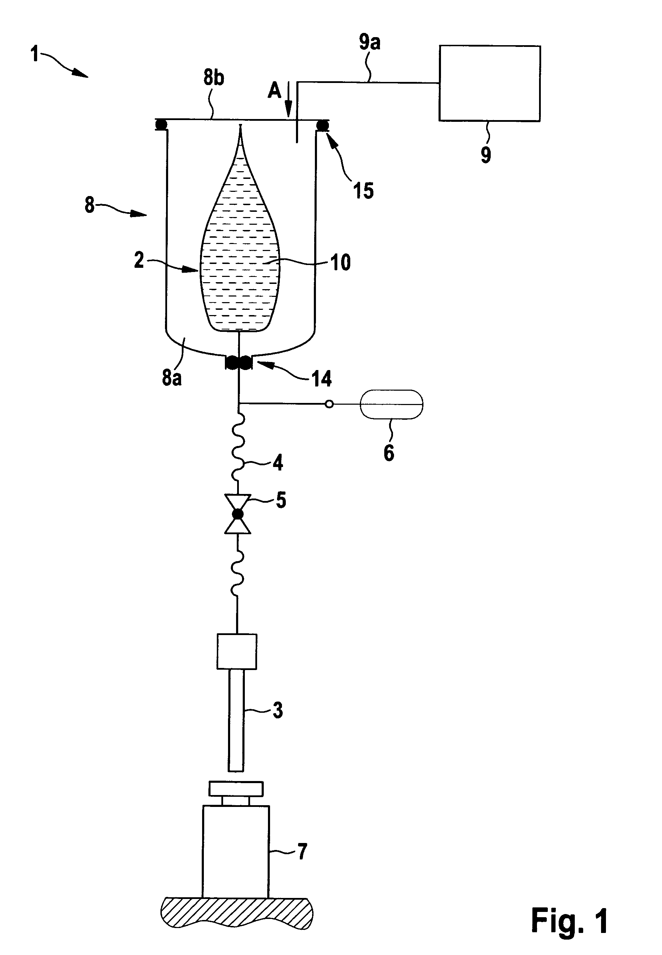

Nachfolgend wird unter Bezugnahme auf Figur 1 eine Füllvorrichtung 1 gemäß einem ersten Ausführungsbeispiel der Erfindung im Detail beschrieben.

Wie in Figur 1 gezeigt, umfasst die Füllvorrichtung 1 des ersten Ausführungsbeispiels einen Beutel 2, welcher mit einem abzufüllenden Produkt 10 gefüllt ist. Das abzufüllende Produkt kann beispielsweise eine pharmazeutische Flüssigkeit sein. Der Beutel 2 ist aus einem flexiblen Material, vorzugsweise einer Kunststofffolie hergestellt. Die Füllvorrichtung 1 umfasst ferner eine Füllnadel 3 sowie einen Schlauch 4. Der Schlauch 4 verbindet den Beutel 2 mit der Füllnadel 3. Dabei ist im Schlauch 4 ein Absperrventil 5 vorgesehen. Das Absperrventil 5 ist als Quetschventil ausgebildet und quetscht von der Außenseite des Schlauches 4 den Durchmesser des Schlauches zusammen, so dass das flüssige Produkt 10 nicht vom Beutel 2 zur Füllnadel 3 strömen kann. Erst wenn das Quetschventil geöffnet wird, ist eine Zufuhr des Produkts 10 zur Füllnadel 3 möglich.

Ferner umfasst die Füllvorrichtung 1 einen Drucksensor 6, welcher am Schlauch 4 angeordnet ist. Der Drucksensor 6 hat die Funktion, einen Druck im Schlauch zu bestimmen. Anhand des Druckes kann dann eine abgegebene Füllmenge über eine vorbestimmte Zeit berechnet werden. Die Füllvorrichtung 1 umfasst ferner ein Druckbehältnis 8, in welchem der Beutel 2 angeordnet ist. Das Druckbehältnis 8 ist mittels eines Deckels 8b verschlossen, so dass ein geschlossener Innenraum 8a im Druckbehältnis 8 definiert ist. Der Beutel 2 ist dabei im Innenraum 8a angeordnet. Zur Abdichtung am Deckel 8b sowie an einer Ausgangsöffnung für den Schlauch 4 sind Dichtungen 14, 15 vorgesehen. Das Druckbehältnis 8 ist somit druckdicht ausgebildet und über eine Leitung 9a mit einem Druckerzeuger 9 verbunden. Der Druckerzeuger 9 ist ein pneumatischer Druckerzeuger, welcher im Innenraum 8a des Druckbehältnisses 8 eine Druckänderung ermöglicht. Insbesondere ermöglicht der Druckerzeuger 9 eine Druckerhöhung im Innenraum 8a, so dass das flüssige Produkt 10 im Beutel 2 bei einem geöffneten Absperrventil 5 durch den Schlauch 4 zur Füllnadel 3 gefördert wird. Wie aus Figur 1 ersichtlich ist, ist unter der Füllnadel 3 ein Fläschchen 7 angeordnet, in welches das Produkt 10 abgefüllt werden muss.

Die erfindungsgemäße Füllvorrichtung 1 ermöglicht nun ein manuelles Abfüllen, indem ein Bediener das Absperrventil 5 in Abhängigkeit vom Füllvolumen im Fläschchen 7 betätigt, oder es ermöglicht auch einen automatischen Abfüllvorgang, wobei eine nicht gezeigte Steuereinheit mit dem Drucksensor 6 und dem Absperrventil 5 verbunden ist und anhand des Druckes eine Abfüllzeit bestimmt, in welcher das Fläschchen 7 befüllt ist. Wenn dies der Fall ist, wird dann automatisch das Absperrventil 5 verschlossen und ein neues leeres Fläschchen 7 unter der Füllnadel 3 angeordnet. Bei einer automatischen Abfüllung wird vorzugsweise eine Einlernkurve in der Steuereinheit hinterlegt, welche eine Funktion der Füllmenge über dem Druck bei einer konstanten Füllzeit ist.

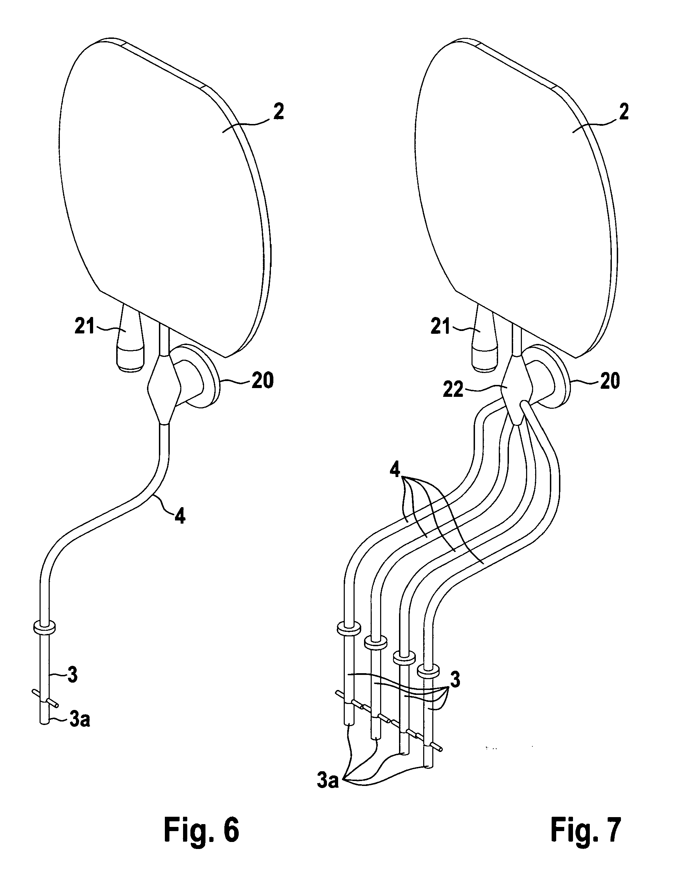

Die erfindungsgemäße Füllvorrichtung hat dabei insbesondere den Vorteil, dass das Produkt 10 nicht in Kontakt mit dem Druckmedium im Druckbehältnis 8 kommt, welches, wie in Figur 1 durch den Pfeil A dargestellt, vom Druckerzeuger 9 in den Innenraum 8a gefördert wird. Mittels des Druckerzeugers 9 ist es ferner möglich, den Druck auf das Produkt 10 sehr gut und einfach zu regeln. Nachdem das gesamte Produkt 10 aus dem Beutel 2 abgefüllt ist, wird der leere Beutel mitsamt dem Schlauch 4 und der Füllnadel 3 entsorgt. Hierdurch ist es insbesondere möglich, dass auf ein aufwendiges Reinigen des Druckbehältnisses 8 sowie der weiteren Teile der Füllvorrichtung 1 verzichtet werden kann. Für einen nächsten Füllvorgang wird einfach ein neuer gefüllter Beutel 2 mit neuem Schlauch 4 und neuer Füllnadel 3 verwendet. Da das Absperrventil 5 nur an der Außenseite des Schlauches 4 angreift, kann dieses ebenfalls wieder verwendet werden. Weiter bevorzugt wird auch der Drucksensor 6 wiederverwendet, wobei eine Verbindung zwischen dem Drucksensor 6 und dem Schlauch derart ausgestaltet ist, dass das Produkt 10 nicht unmittelbar mit dem Drucksensor 6 in Kontakt kommt. Dies kann beispielsweise durch Vorsehen einer Membran 20 erreicht werden, welche das Produkt 10 vom Drucksensor 6 trennt. Eine derartige Ausgestaltung ist in Figur 6 gezeigt. Dadurch kann die erfindungsgemäße Füllvorrichtung 1 noch kostengünstiger bereitgestellt werden, da auch der Drucksensor 6 wiederverwendet werden kann. Alternativ ist auch ein Nachfüllen des Beutels möglich. Dazu ist vorzugsweise eine Niveaukontrolle vorhanden, um bei minimalem Füllstand den Beutel nachzufüllen.

Ein weiterer Vorteil der erfindungsgemäßen Füllvorrichtung ist, dass diese als vorsterilisiertes System lieferbar ist, so dass ein Kunde keine Teile vor einem Abfüllvorgang autoklavieren muss. Ferner ist es möglich, in Abhängigkeit von dem abzufüllenden Produkt 10 jeweils einen individuell ausgelegten Schlauch 4 bzw. eine individuell ausgelegte Füllnadel 3 vorzusehen, beispielsweise, wenn die Produkte verschiedene Viskositäten aufweisen.

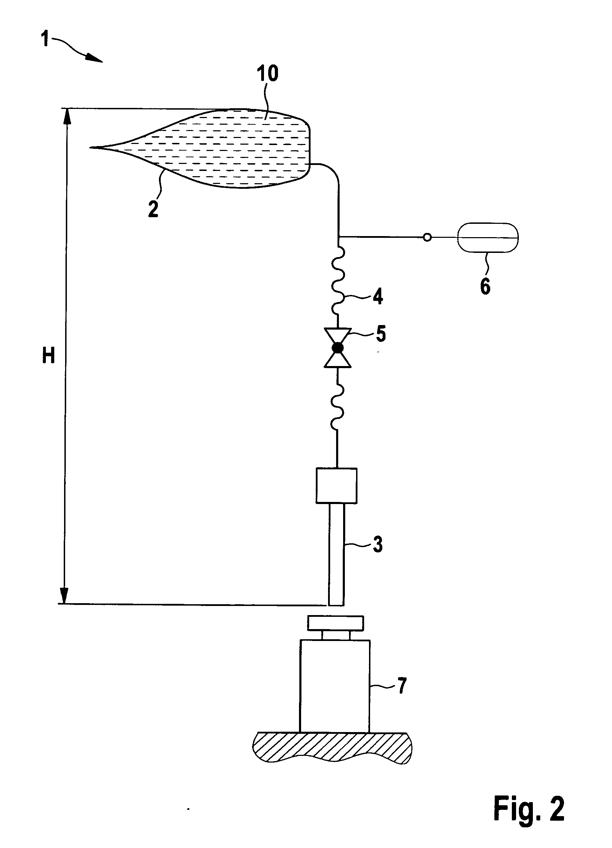

Figur 2 zeigt eine Füllvorrichtung 1 gemäß einem zweiten Ausführungsbeispiel, wobei gleiche bzw. funktional gleiche Teile mit den gleichen Bezugszeichen wie im ersten Ausführungsbeispiel bezeichnet sind. Im Unterschied zu der Füllvorrichtung des ersten Ausführungsbeispiels weist die Füllvorrichtung des zweiten Ausführungsbeispiels kein Druckbehältnis 8 auf. Wie aus Figur 2 ersichtlich ist, ist ein Beutel 2 mit einer vorbestimmten Höhe H über einem Ende der Füllnadel 3 angeordnet. Der Druck auf das Produkt 10 im Beutel 2 wird somit lediglich über die statische Höhe H zwischen einem Füllspiegel des Beutels 2 und dem Austritt aus der Füllnadel 3 erzeugt. Diese Füllvorrichtung 1 des zweiten Ausführungsbeispiels weist einen sehr einfachen Aufbau auf, und insbesondere können Beutel mit beliebigen Größen bei der Füllvorrichtung 1 des zweiten Ausführungsbeispiels verwendet werden, da keine Formatbeschränkungen, beispielsweise durch ein Druckbehältnis, gegeben sind.

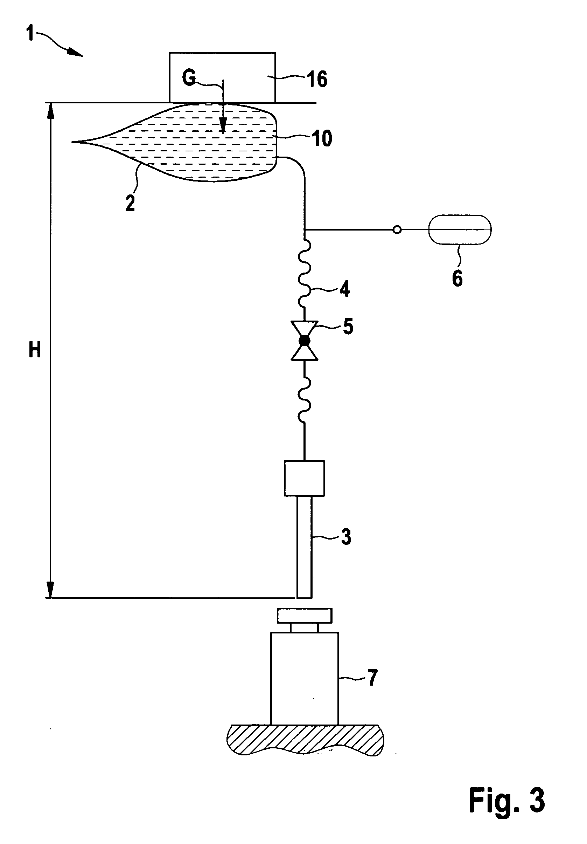

Figur 3 zeigt eine Füllvorrichtung 1 gemäß einem dritten Ausführungsbeispiel der Erfindung, wobei gleiche bzw. funktional gleiche Teile mit den gleichen Bezugszeichen wie in den vorhergehenden Ausführungsbeispielen bezeichnet sind.

Das dritte Ausführungsbeispiel entspricht im Wesentlichen dem zweiten Ausführungsbeispiel, wobei im Unterschied zum zweiten Ausführungsbeispiel zusätzlich noch ein Gewicht 16 auf dem Beutel 2 angeordnet ist. Das Gewicht 16 wirkt mit einer zusätzlichen Gewichtskraft G auf das Produkt 10 im Beutel, wodurch ein zusätzlicher Druck auf das Produkt 10 erzeugt wird. Somit ist eine höhere Ausbringung der Füllvorrichtung 1 möglich. Dabei ist der Aufbau der Füllvorrichtung 1 des dritten Ausführungsbeispiels ebenfalls sehr einfach.

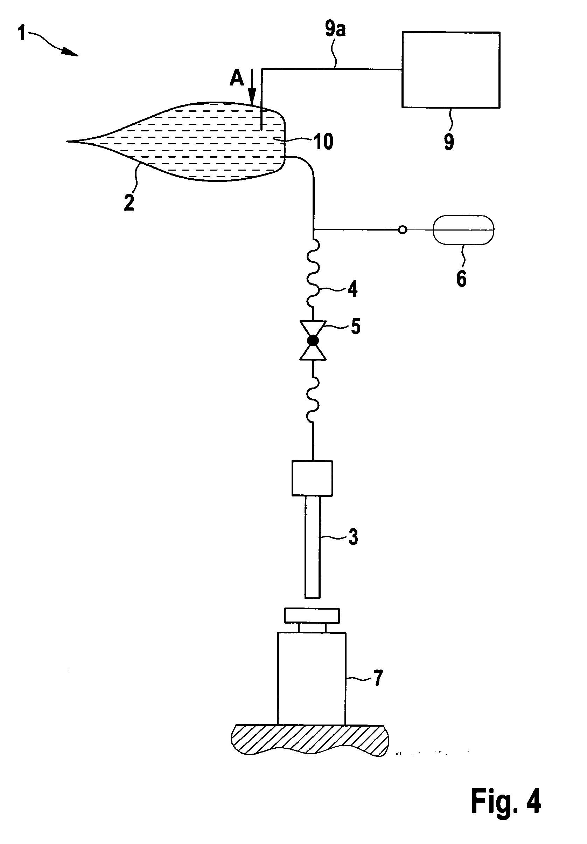

Figur 4 zeigt eine Füllvorrichtung 1 gemäß einem vierten Ausführungsbeispiel der Erfindung, wobei gleiche bzw. funktional gleiche Teile mit den gleichen Bezugszeichen wie in den vorhergehenden Ausführungsbeispielen bezeichnet sind.

Die Füllvorrichtung 1 des vierten Ausführungsbeispiels umfasst ebenfalls einen Druckerzeuger 9, welcher jedoch über eine Leitung 9a mit dem Inneren des Beutels 2 verbunden ist. Somit wird Druck auf das Produkt 10 durch Zuführen eines Gases in das Innere des Beutels 2 erzeugt. Da das Gas hierbei mit dem Produkt 10 in Kontakt kommt, muss das Gas als steriles Gas eingebracht werden, wenn es sich um ein pharmazeutisches Produkt 10 handelt. Auch bei der Füllvorrichtung des vierten Ausführungsbeispiels kann auf ein separates Druckbehältnis, in welchem der Beutel 2 angeordnet ist, verzichtet werden und es ist jede Beutelgröße möglich.

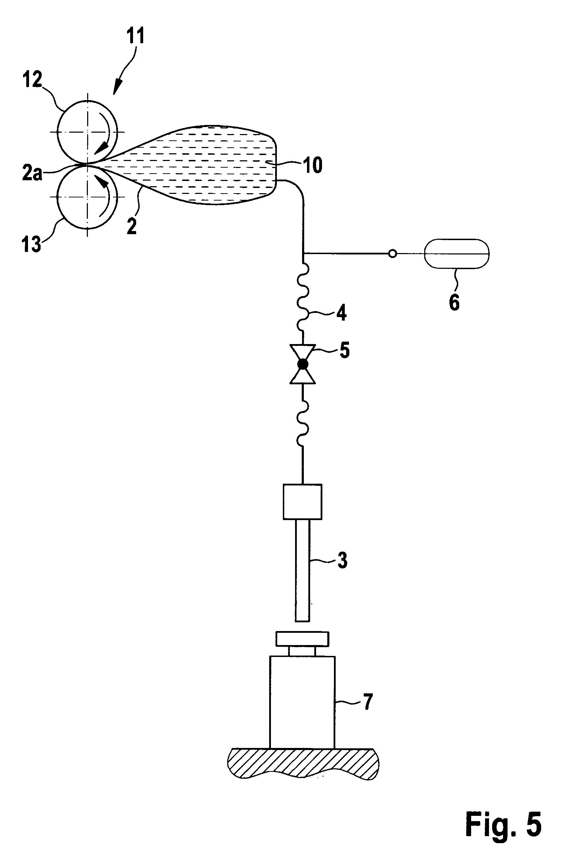

Figur 5 zeigt eine Füllvorrichtung 1 gemäß einem fünften Ausführungsbeispiel der Erfindung, wobei gleiche bzw. funktional gleiche Teile mit den gleichen Bezugszeichen wie in den vorhergehenden Ausführungsbeispielen bezeichnet sind.

Wie aus Figur 5 ersichtlich ist, umfasst die Füllvorrichtung 1 des fünften Ausführungsbeispiels eine Auspresseinheit 11, welche durch Drücken auf den flexiblen Beutel 2 einen Druck auf das Produkt 10 ausübt und dieses aus dem Beutel 2 auspresst. Die Auspresseinheit 11 umfasst eine erste Rolle 12 und eine zweite Rolle 13, welche nebeneinander angeordnet sind und zwischen sich ein Ende 2a des Beutels 2 einklemmen. Durch Rotation der beiden Rollen 12, 13 wird das Ende 2a des Beutels 2 zwischen die beiden Rollen gezogen und das Produkt 10 aus dem Beutel 2 in den Schlauch 4 ausgepresst. Durch Regelung der Drehgeschwindigkeit der Rollen 12, 13 kann dabei eine Druckhöhe auf das Produkt geregelt werden.

Es sei angemerkt, dass es alternativ auch möglich ist, dass das Ende 2a des Beutels 2 an einer der Rollen befestigt wird und bei Drehung dieser Rolle dieses aufgewickelt wird. Die zweite Rolle dient dabei als abstützende Rolle, welche das Produkt 10 aus dem Beutel 2 herausdrückt.

Somit weist die Füllvorrichtung 1 des fünften Ausführungsbeispiels ebenfalls einen einfachen und kostengünstigen Aufbau auf, ohne dass ein zusätzlicher Druckerzeuger und ein zusätzliches Druckmedium notwendig ist.

Der in Figur 6 gezeigte Beutel 2 mit dem Schlauch 4, an welchem die Membran 20 angeordnet ist, kann dabei in allen im vorherigen beschriebenen Füllvorrichtungen 1 verwendet werden. Vorzugsweise ist der Schlauch 4 dabei einstückig mit dem Beutel 2 gebildet. Um vor dem Abfüllvorgang ein unbeabsichtigtes Herauslaufen des Produkts 10 aus der Füllnadel 3 zu vermeiden, ist am Ende der Füllnadel 3 eine Kappe 3a angeordnet, welche vor dem Abfüllvorgang einfach abgezogen werden kann. Das Bezugszeichen 21 bezeichnet einen Stutzen am Beutel 2, über welchen eine Befüllung des Beutels 2 erfolgt. Am Stutzen 21 kann ein größerer Beutel oder Tank angeschlossen werden, so dass eine komplette Charge eines Produkts über den Beutel 2 abgefüllt werden und dann der Beutel 2 entsorgt werden kann. Figur 7 zeigt dabei eine alternative Schlauchanordnung, bei der eine Vielzahl von Schläuchen 4 mit einer Vielzahl von Füllnadeln 3 vorgesehen sind. Die Schläuche 4 zweigen dabei von einem Verzweigungselement 22 ab. In diesem Ausführungsbeispiel sind vier abzweigende Schläuche 4 vorgesehen. Hierdurch wird insbesondere die Ausbringungsmenge der Füllvorrichtung vergrößert. Wie weiter aus Figur 7 ersichtlich ist, ist am Verzweigungselement 22 auch die Membran 20 angeordnet, an welcher ein Drucksensor 6 befestigbar ist. Hierzu weist das Verzweigungselement 22 ein gewisses Innenvolumen auf, um problemlos die Vielzahl der Schläuche sowie die Membran 20 anzuordnen.

Die erfindungsgemäße Füllvorrichtung 1 ermöglicht es somit, dass im Vergleich mit dem Stand der Technik auf einen aufwendigen Aufbau mit einem Produktbehälter für das abzufüllende Produkt auf Produktfilter und eine Drossel im Schlauch verzichtet werden kann. Somit ergibt sich erfindungsgemäß ein deutlich vereinfachter Aufbau. Ferner ist es erfindungsgemäß nicht mehr notwendig, eine Reinigung des Produktbehälters sowie weitere Teile vorzusehen, so dass insbesondere Stillstandszeiten von Füllmaschinen signifikant reduziert werden können.

## Patentansprüche

1. Füllvorrichtung zum Abfüllen einer definierten Menge eines Produkts (10) in einen Behälter (7), umfassend
- einen Beutel (2) aus einem flexiblen Material, welches mit dem abzufüllenden Produkt (10) gefüllt ist,
- eine Füllnadel (3),
- einen Schlauch (4), welcher den Beutel (2) mit der Füllnadel (3) verbindet,
- ein Absperrventil (5), welches am Schlauch (4) angeordnet ist, um einen Durchfluss durch den Schlauch (4) freizugeben oder abzusperren,
- wobei der Beutel (2), die Füllnadel (3) und der Schlauch (4) eine Baugruppe bilden, welche als Einwegartikel ausgebildet ist,
- ein geschlossenes Druckbehältnis (8), in welchem der Beutel (2) angeordnet ist, und
- eine Druckerzeugungseinheit (9),
- wobei ein Innenbereich (8a) des Druckbehältnisses (8) mit einem Druck beaufschlagbar ist, um den Beutel (2) unter Druck zu setzen, wobei der Druck mittels der Druckerzeugungseinheit (9) erzeugt wird.

2. Füllvorrichtung nach Anspruch 1, ferner umfassend einen Drucksensor (6), welcher am Schlauch (4) angeordnet ist, um einen Druck im Schlauch (4) zu erfassen.

3. Füllvorrichtung nach Anspruch 2, **dadurch gekennzeichnet, dass** am Schlauch (4) eine Membran (20) angeordnet ist, deren erste Membranfläche mit dem im Schlauch geförderten Produkt (10) in Kontakt ist und deren zweite Membranfläche mit dem Drucksensor (6) in Kontakt ist, wobei die Membran (20) eine Kontaminierung des Drucksensors (6) mit dem Produkt (10) verhindert.

4. Füllvorrichtung nach einem der vorhergehenden Ansprüche, **dadurch gekennzeichnet, dass** das Absperrventil (5) eine Schlauchquetsche ist, welche den Schlauch (4) von außen quetscht.

5. Füllvorrichtung nach einem der vorhergehenden Ansprüche, ferner umfassend ein Verzweigungselement (22), welches im Schlauch (4) angeordnet ist und von welchem mehrere einzelne Schläuche aus abgehen, um gleichzeitig mehrere Behälter (7) zu befüllen.

6. Zur Abfüllung von pharmazeutischen Produkten ausgelegte Füllmaschine mit einer Füllvorrichtung nach einem der vorhergehenden Ansprüche und einer Behälterzufuhreinrichtung zum Zuführen von Behältern (7), in welche das Produkt (10) abgefüllt wird.

## Claims

1. Filling apparatus for filling a defined quantity of a product (10) into a container (7), comprising
- a bag (2) made of flexible material, which is filled with the product (10) to be filled,
- a filling needle (3),
- a flexible tube (4) which connects the bag (2) to the filling needle (3),
- a shut-off valve (5) which is arranged on the flexible tube (4) in order to enable or shut off a through-flow through the flexible tube (4),
- wherein the bag (2), the filling needle (3) and the flexible tube (4) form a subassembly which is in the form of a disposable article,
- a closed pressure vessel (8) in which the bag (2) is arranged, and
- a pressure-generating unit (9),
- wherein an internal region (8a) of the pressure vessel (8) can be subjected to a pressure in order to place the bag (2) under pressure, wherein the pressure is generated by means of the pressure-generating unit (9).

2. Filling apparatus according to Claim 1, further comprising a pressure sensor (6) which is arranged on the flexible tube (4) in order to sense a pressure in the flexible tube (4).

3. Filling apparatus according to Claim 2, **characterized in that** a membrane (20) is arranged on the flexible tube (4), the first membrane surface of said membrane being in contact with the product (10) conveyed in the flexible tube and the second membrane surface being in contact with the pressure sensor (6), wherein the membrane (20) prevents contamination of the pressure sensor (6) with the product (10).

4. Filling apparatus according to one of the preceding claims, **characterized in that** the shut-off valve (5) is a tube pincher which pinches the flexible tube (4) from outside.

5. Filling apparatus according to one of the preceding claims, further comprising a branching element (22) which is arranged in the flexible tube (4) and from which a plurality of individual flexible tubes proceed, in order to fill a plurality of containers (7) simultaneously.

6. Filling machine designed for filling pharmaceutical products, having a filling apparatus according to one of the preceding claims and a container supplying device for supplying containers (7) into which the product (10) is filled.

## Revendications

1. Dispositif de remplissage pour verser une quantité définie d'un produit (10) dans un récipient (7), comprenant :
- un sachet (2) en matériau flexible, qui est rempli avec le produit à verser (10),
- une aiguille de remplissage (3),
- un tuyau flexible (4) qui relie le sachet (2) à l'aiguille de remplissage (3),
- une vanne d'arrêt (5) qui est disposée sur le tuyau flexible (4) pour libérer ou bloquer la circulation de fluide à travers le tuyau flexible (4),
- le sachet (2), l'aiguille de remplissage (3) et le tuyau flexible (4) formant un module qui est réalisé sous forme d'article à usage unique,
- un récipient sous pression fermé (8), dans lequel est disposé le sachet (2), et
- une unité de génération de pression (9),
- une région interne (8a) du récipient sous pression (8) pouvant être sollicitée en pression, afin de mettre le sachet (2) sous pression, la pression étant produite à l'aide de l'unité de génération de pression (9).

2. Dispositif de remplissage selon la revendication 1, comprenant en outre un capteur de pression (6) qui est disposé sur le tuyau flexible (4), afin de détecter une pression dans le tuyau flexible (4).

3. Dispositif de remplissage selon la revendication 2, **caractérisé en ce qu'**une membrane (20) est disposée sur le tuyau flexible (4), la première surface de la membrane étant en contact avec le produit (10) refoulé dans le tuyau flexible, et la deuxième surface de la membrane étant en contact avec le capteur de pression (6), la membrane (20) empêchant une contamination du capteur de pression (6) par le produit (10).

4. Dispositif de remplissage selon l'une quelconque des revendications précédentes, **caractérisé en ce que** la vanne d'arrêt (5) est un pinçage de tuyau qui comprime le tuyau flexible (4) depuis l'extérieur.

5. Dispositif de remplissage selon l'une quelconque des revendications précédentes, comprenant en outre un élément de branchement (22) qui est disposé dans le tuyau flexible (4) et duquel partent plusieurs tuyaux flexibles individuels, afin de remplir simultanément plusieurs récipients (7).

6. Machine de remplissage conçue pour le remplissage avec des produits pharmaceutiques, comprenant un dispositif de remplissage selon l'une quelconque des revendications précédentes et un dispositif d'apport de récipients pour acheminer des récipients (7) dans lesquels est versé le produit (10).
